# EUROPEAN PATENT APPLICATION

(11) **EP 2 471 385 A1**
(43) Date of publication of application: **04.07.2012**
(21) Application number: 10197295.8
(22) Date of filing: 29.12.2010
(51) Int. Cl.: A23L 1/30, A61K 31/23, A61K 9/00, A61K 9/16

(54) **Cetyl myristate and/or cetyl palmitate suspension formulations**

(71) Applicant: Deva Holding Anonim Sirketi, Kucukcekmece/Istanbul (TR)
(72) Inventor: Haas, Philipp Daniel, 34303, ISTANBUL (TR); Firat, Omer Faruk, 34303, ISTANBUL (TR); Kandemir, Levent, 34303, ISTANBUL (TR); Koc, Fikret, 34303, ISTANBUL (TR); Sivasligil, Ramazan, 34303, ISTANBUL (TR)

(57) **Abstract**

This invention is related to spray drying or melt granulation or dissolving or dispersing treatment of cetyl myristate or cetyl palmitate or combination of cetyl myristate and cetyl palmitate.

## Description

### Technical Field

This invention is related to spray drying or melt granulation or dissolving or dispersing treatment of cetyl myristate or cetyl palmitate or combination of cetyl myristate and cetyl palmitate.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process preparing a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention embraces spray drying or melt granulation or dispersion or dissolution treatment of cetyl myristate or cetyl palmitate or combination of cetyl myristate and cetyl palmitate.

### Technical Problem

Cetyl myristate or cetyl palmitate or combination of cetyl myristate and cetyl palmitate can be used as active pharmaceutical ingredients (API) in pharmaceutical formulations. However, both cetyl myristate and cetyl palmitate are waxy ingredients and in formulation stage they are adhered onto the surfaces of formulation equipments. It gives rise that granulation of API's is hardly possible.

Another problem is observed in respect of dosing, since cetyl palmitate is approximately used 20 times less than cetyl myristate in pharmaceutical or dietary supplement formulations. It leads to dose uniformity problems. Uniformity of low dose solid dosage forms is impacted by granulation methods and causes poor blend homogeneity due to agglomeration and/or segregation.

### Solution to Problem

It is invented that sticking and dose uniformity problems are solved by use of melt granulation or spray drying techniques.

It is also invented that sticking and dose uniformity problem is solved by dissolving or dispersing the cetyl palmitate or cetyl myristate or combination of cetyl palmitate and cetyl myristate and then dispersed or dissolved API(s) are treated onto surface of API(s) or excipients or mixtures of API(s) and excipients.

### Description of embodiments

One aspect of this invention is to provide spray dried granules of cetyl myristate or cetyl palmitate or combination of cetyl myristate and cetyl palmitate.

Spray drying provides eligible and elegant powder of cetyl myristate or cetyl palmitate or combination of cetyl myristate and cetyl palmitate. Spray drying leads to non-stick and easily flowable granules having desired level of dose uniformity. Dose uniformity is essentially important point especially for low dose active ingredients. In accordance with this invention cetyl palmitate is used about 20 times less than cetyl myristate, hence dose uniformity problem is unavoidable. Since spray drying involves converting the atomized liquid droplets into dry powders by hot air, the spray-dried powder particles are homogeneous.

Spray drying is also more economic than other processes since it produces dry powder directly from a liquid and eliminates drying, additional drying ,milling and such processing. Thus it leads to saving time, money and energy.

In another aspect, this invention relates to the preparation of pharmaceutical powder compositions compressible into tablets or fillable into capsules.

The spray-dried material can also be blended with the active ingredient or excipients such as lubricants and direct compression agent to obtain tablets or capsules.

In another aspect, this invention relates to preparing of slurry or solution for spray drying. In preparation stage, suitable excipients such as binders and disintegrants may be combined with the active agents in the slurry or solution. The slurry or solution of cetyl myristate or cetyl palmitate or combination of cetyl myristate and cetyl palmitate can be directly spray dried or they can be combined with one or more excipients before spray drying.

According to this invention, the spray dried powder comprising the cetyl myristate or cetyl palmitate or combination of cetyl myristate and cetyl palmitate; or spray dried powder comprising cetyl myristate or cetyl palmitate or combination of cetyl myristate and cetyl palmitate and one or more excipients can be combined with additional excipient or mixtures of excipients.

Another aspect of the present invention is that the spray dried powder of cetyl myristate or cetyl palmitate or combination of cetyl myristate and cetyl palmitate has excellent flowability.

Another aspect of the present invention is that spray dried powder of cetyl myristate or cetyl palmitate or combination of cetyl myristate and cetyl palmitate are evenly distributed.

Another aspect of the present invention is that spray dried powder of cetyl myristate or cetyl palmitate or combination of cetyl myristate and cetyl palmitate can be directly compressible.

In accordance with this invention slurry or solution are stirred to 30°C or beyond, preferably 40°C or beyond, and it is remained during the spray drying in order to prepare suitable fluid material.

Additional excipients or mixtures can be used to accelerate the process and reduce the melting points.

The slurry or solution of cetyl myristate or cetyl palmitate or combination of cetyl myristate and cetyl palmitate may also be spray dried without excipient or mixtures of excipients.

The slurry or solution of cetyl myristate or cetyl palmitate or combination of cetyl myristate and cetyl palmitate or admixture thereof with an excipient or mixtures of excipients can be spray dried by the use of conventional spray drying apparatuses like vertical spray dryer or fluid spray dryer. In this invention spray-drying is done using a Buchi Mini Spray Dryer B-290.

Inlet or ambient temperature of apparatuses is 25°C or higher. Said temperature is not higher than 100°C.

In combination of cetyl myristate and cetyl palmitate:each active ingredients can be spry dried separately and then they can be combined or both active ingredients can be spray dried together or both API's can be spray dried with an excipient or mixtures of excipients.

Another aim of this invention is to provide granules of cetyl myristate or cetyl palmitate or combination of cetyl myristate and cetyl palmitate obtained by melt granulation.

Melt granulation brings numerous advantages. It does not require using of solvent or water and saves time since drying steps are eliminated. There are also no requirements for the compressibility of active ingredients and procedure leads to continuous process.

In accordance with this invention it is also invented that uniform dispersion of low dose active ingredient is provided through melt granulation. Additionally, pharmaceutical or dietary supplement compositions of cetyl miyristate and cetyl palmitate or cetyl myristate and cetyl palmitate combination have eligible disintegrating properties due to melt granulation.

According to this invention melt granulation process comprises the steps of: (a) forming a melt mixture by mixing a molten cetyl myristate or cetyl palmitate or combination of cetyl myristate and cetyl palmitate, optionally with one or more excipients and (b) forming the melt mixture into solidified melt granules.

Preferably, step (a) of the process of the present invention takes place in a melt extruder. Melt granulation also can be carried out such as fluidised bed (FB) Mini-Glatt, high-shear mixer (Rotolab®) equipped with an electric heated jacket and the like.

Melting is executed at a temperature higher than 30°C, preferably higher than 40°C. Melting is executed at a temperature not higher than 150°C. For instance, the temperature of the extruder barrel or melting is 50°C. Melting material may be only cetyl myristate or cetyl palmitate or combination of cetyl myristate and cetyl palmitate; or an excipient or mixtures of excipients may be added thereto. If excipient or mixtures of excipients, such as melt granulating agent, are used, said mixture is heated to a temperature greater than the melting point of said excipient or mixture of excipients.

Melt granulating agent is, but not limited to, Poloxamer, Polyethylene glycol, Beeswax, Glyceryl behenate, Glyceryl monostearate, Glyceryl palmitostearate, Glyceryl stearate, Glyceryl Monooleate,Hydrogenated castor oil, Microcrystalline wax, Paraffin wax, Stearic acid, Stearic alcohol, and polyethylene glycol,cetostearyl alcohol, stearyl alcohol, cetyl alcohol, myristyl alcohol and lauryl alcohol, hydrogenated vegetable oil, tristearin, tripalmitin, cetyl ester wax, mixture thereof and the like.

The melt granules may be combined with the extra-granular component and blended so as to form a uniform mixture of ingredients. Following apparatuses may be utilized for operation: Ribbon Blender, IBC Blender, V-Blender, Plough Blender and the like.

In another aspect, it is invented that when cetyl myristate or cetyl palmitate or combination of cetyl myristate and cetyl palmitate is dissolved or dispersed in a solution or suspension and then treated onto the particles of active ingredient(s) or excipient(s) or mixtures thereof, dose uniformity is elegantly provided. Treatment may be in the form of adhering, adsorption etc.

In accordance with this invention one of the following non restrictive examples can be applied:

a) Cetyl Myristate and optionally excipient or excipients are dissolved or dispersed and treated onto the particles of cetyl palmitate and optionally excipient(s)

b) Cetyl Palmitate and optionally excipient or excipients are dissolved or dispersed and treated onto the particles of cetyl myristate and optionally excipient(s)

c) Cetyl Palmitate and cetyl myristate and optionally excipient or mixture of excipients are dissolved or dispersed and treated onto the particles of excipient(s)

d) Cetyl Myristate and optionally excipient or mixture of excipients are dissolved or dispersed and treated onto the particles of excipient(s)

e) Cetyl Palmitate and optionally excipient or mixture of excipients are dissolved or dispersed and treated onto the particles of excipient(s)

Said solution or dispersion comprising;

(i) an inorganic acid or mixtures of inorganic acids or,

(ii) an organic acid or mixtures of organic acids or,

(iii) mixtures of organic acid or organic acids and inorganic acid or inorganic acids and

(v) an organic solvent or mixtures of organic solvents and

(vi) optionally de-ionized water and,

(vii) optionally binder or binders.

Organic solvents used in preparation of solution or dispersion are but not limited to, alcohols such as methanol, ethanol, propyl alcohol, isopropyl alcohol, 2-methyl-1-propanol etc.; ketones such as acetone, methyl ethyl ketone, methyl isobutyl ketone etc.; esters such as methyl acetate, ethyl acetate, propyl acetate, isopropyl acetate, ethyl formate etc.; hydrocarbons such as heptane; halogenated hydrocarbons such as dichloromethane.

Organic acids are but not limited to formic acid, acetic acid, fumaric acid, tartaric acid, maleic acid, citric acid, succinic acid, malic acid, methanesulfonic acid, p-toluenesulfonic acid.

Inorganic acid is hydrochloric acid in the form of solution in water.

In accordance with present invention, the pharmaceutical or dietary supplement composition comprises cetyl myristate or cetyl palmitate or cetyl palmitate and cetyl myristate combination, disintegrant, lubricant, binder, filler and the like.

Disintegrants are, but not limited to, alginic acid, chitosan, pectins, cation exchange resins, magnesium silicates, aluminium silicates, mixtures thereof and the like.

Lubricants/Glidants are, but not limited to, magnesium stearate, calcium stearate, hydrogenated castor oil, glyceryl behenate, glyceryl monostearate, glyceryl palmitostearate, leucine, mineral oil, light mineral oil, myristic acid, palmitic acid, polyethylene glycol, potassium benzoate, sodium benzoate, sodium lauryl sulfate, sodium stearyl fumarate, stearic acid, talc, hydrogenated vegetable oil, zinc stearate, magnesium lauryl sulphate,sodium stearyl fumarate, polyethylene glycol, stearic acid, colloidal silicon dioxide or mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose, tragacanth, sorbitol, acacia, alginates, carrageenan, xanthan gum, locust bean gum and gum arabic, waxes, polyacrylamide, mixtures thereof, and the like.

Diluents/fillers are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

According to this invention, spray drying, melt granulation and treatment of solution or dispersion are preferably applied for a pharmaceutical or dietary supplement composition wherein cetyl myristate is 333.3 mg and cetyl palmitate is 16.7 mg. In pharmaceutical or dietary supplement formulations, cetyl miyristate or cetyl palmitate or cetyl myristate or cetyl palmitate combination is present from 40% to 95% by weight of pharmaceutical composition

### Example 1

Cetyl myristate or cetyl palmitate or combination of cetyl myristate and cetyl palmitate are stirred in a stainless steel container equipped with a suitable stirrer at a temperature of 55°C and said temperature is remained during the spray drying . A fluidized spray dryer is used. The slurry or solution is sprayed through a fluid nozzle. The fluid bed inlet temperature is 30°C. The spray dried of cetyl myristate or cetyl palmitate or combination of cetyl myristate and cetyl palmitate are blended with excipients.

### Example 2

35 mg of (hydroxypropyl)methyl cellulose is dissolved in 1000 ml of purified water and 42 mg of lactose, 333.3 mg of cetyl myristate and 16.70 mg of cetyl palmitate are added and stirred to 52°C to produce a suspension. The suspension is mixed for 10 minutes. Then, the suspension is spray-dried using a mini spray dryer (Buchi 190) under the condition of an inlet temperature of 30-40°C to produce spray-dried granules.

### Example 3

40 mg of polyvinylpyrrolidone and 60m g of Polysorbate 80 are dissolved in 1000 ml of purified water and said mixture is stirred to 47°C and 70 mg of lactose and 333.3 mg of cetyl myristate and 16.70 mg of cetyl palmitate are added thereto and mixed to produce a suspension. Then, the suspension is spray-dried using a mini spray dryer (Buchi 190) under the condition of an inlet temperature of 30-40 °C to produce spray-dried granules.

### Example 4

Cetyl myristate, cetyl palmitate, PEG 6000 and lactose are mixed by fluidising. Air temperature is raised to 70-80°C. Once the product temperature reached 50-60°C, the timing of the kneading phase started. Finally inlet air temperature is decreased to 20°C and it is remained until the product temperature reached 25°C. At the end of the granulation process, the granules are sieved.

### Example 5

Cetyl myristate or cetyl palmitate or combination of cetyl myristate and cetyl palmitate, optionally with other excipients are mixed and then heated to temperature of 40°C to 50°C in an extruder. The molten mass is cooled. The molten mixture is solidified and the solid mass is milled. Colloidal silicon dioxide, magnesium stearate, stearic acid, dicalcium phosphate and microcrystalline cellulose are blended with the granular composition. The dry blended material is loaded to a rotary tabletting machine and compressed into tablets.

### Example 6

**Table 1**

| **FORMULATION OBTAINED BY MELT GRANULATION** | |
|---|---|
| **Ingredients** | **mg** |
| Cetyl Myristate | 333.3 |
| Cetyl Palmitate | 16.7 |
| Polyethylene glycol | 25 |
| Croscarmellose sodium | 10 |
| Microcrystalline cellulose | 20 |
| Colloidal silicon dioxide | 5 |
| Magnesium stearate | 5 |
| Stearic acid | 5 |
| Di-calcium phosphate | 20 |
| **Total** | **440** |

### Example 7

Treatment onto the particles is carried out through following steps ;

(a) Organic solvent, de-ionized water and inorganic acid are mixed in a container.

(b) Binder or mixtures thereof are added to the mixture obtained from step (a) and continuously stirred.

(c) Cetyl palmitate is added to mix obtained from step b and continuously stirred to 50°C and remain 50°C during the operation.

(e) Cetyl myristate and excipients comprising mixtures of diluents and disintegrants are layed on the bed.

(f)The prepared material obtained from (c) is sprayed onto the mixture of cetyl myristate and excipients.

## Claims

1. Spray-dried granules comprising cetyl myristate or cetyl palmitate or combination of cetyl myristate and cetyl palmitate and preferably an excipient or mixtures of excipients.

2. Spray drying, as claimed in claim 1, is performed from a slurry or solution.

3. Slurry or solution, as claimed in claim 2, comprising cetyl myristate or cetyl palmitate or combination of cetyl myristate and cetyl palmitate; or comprising cetyl myristate or cetyl palmitate or combination of cetyl myristate and cetyl palmitate and one or more excipients.

4. The spray dried powder comprising the cetyl myristate or cetyl palmitate or combination of cetyl myristate and cetyl palmitate; or spray dried powder comprising cetyl myristate or cetyl palmitate or combination of cetyl myristate and cetyl palmitate and one or more excipients can be combined additional excipient or mixtures of excipients.

5. Slurry or solution, as claimed in claim 2, is stirred to 30°C or beyond, preferably 40°C or beyond, and said temperature is remained during the spray drying.

6. Melt granulate comprising cetyl myristate or cetyl palmitate or combination of cetyl myristate and cetyl palmitate and optionally an excipient or mixtures of excipients.

7. Melt granulate, as claimed in claim 6, is obtained by a process comprising the steps of: (a) forming a melt mixture by mixing a cetyl myristate or cetyl palmitate or combination of cetyl myristate and cetyl palmitate and optionally with one or more excipients; and (b) forming the melt mixture into solidified melt granules.

8. Melting, as claimed in claim 7, is executed at a temperature higher than 30°C, preferably higher than 40°C through suitable apparatus.

9. Excipient or mixtures of excipients, as claimed in claim 8, is heated to a temperature higher than the melting point of said excipient or mixture of excipients.

10. A pharmaceutical or dietary supplement formulation of cetyl myristate or cetyl palmitate or combination of cetyl myristate and cetyl palmitate **characterized in that a)** Cetyl Myristate and optionally excipient or excipients are dissolved or dispersed and treated onto the particles of cetyl palmitate and optionally excipient or mixtures of excipients or; **b)** Cetyl Palmitate and optionally excipient or excipients are dissolved or dispersed and treated onto the particles of cetyl myristate and optionally excipient or mixtures of excipients or; **c)** Cetyl Palmitate and cetyl myristate and optionally excipient or mixture of excipients are dissolved or dispersed and treated onto the particles of excipient or mixtures of excipients or; **d)** Cetyl Myristate and optionally excipient or mixture of excipients are dissolved or dispersed and treated onto the particles of excipient or mixtures of excipients or; **e)** Cetyl Palmitate and optionally excipient or mixture of excipients are dissolved or dispersed and treated onto the particles of excipient or mixtures of excipients

11. Solution or dispersion, as claimed in claim 9, comprises; **(i)** an inorganic acid or mixtures of inorganic acids or; **(ii)** an organic acid or mixtures of organic acids or; **(iii)** mixtures of organic acid or organic acids and inorganic acid or inorganic acids and **(v)** an organic solvent or mixtures of organic solvents and **(vi)** optionally de-ionized water and **(vii)** optionally binder or binders.

12. According to preceding claims, spray drying, melt granulation and treatment of solution or dispersion are applied for a pharmaceutical or dietary supplement composition wherein cetyl myristate is 333.3 mg and cetyl palmitate is 16.7 mg.

13. Producing methods, as claimed in preceding claims, are applied to produce pharmaceutical or dietary supplement compositions of cetyl miyristate or cetyl palmitate or cetyl myristate or cetyl palmitate combination wherein cetyl miyristate or cetyl palmitate or cetyl myristate or cetyl palmitate combination is present from 40% to 95% by weight.
